# EUROPEAN PATENT APPLICATION

(11) **EP 4 474 385 A1**
(43) Date of publication of application: **11.12.2024**
(21) Application number: 23382576.9
(22) Date of filing: 09.06.2023
(51) Int. Cl.: C07F 15/02, C07F 15/06

(54) **PROCESS FOR THE PHOTOCATALYTIC OXIDATION OF ALKANES AND AROMATIC HYDROCARBONS**

(71) Applicant: Consejo Superior de Investigaciones Cientificas, 28006 Madrid (ES); Universitat De Girona, 17003 Girona (ES)
(72) Inventor: Teixidor i Bombardó, Francesc, Barcelona (ES); Viñas i Teixidor, Clara, Barcelona (ES); Núñez Aguilera, Rosario, Barcelona (ES); Guerrero Troyano, Isabel, Barcelona (ES); Romero García, Isabel, Barcelona (ES)
(74) Representative: Pons

(57) **Abstract**

The present invention relates to a process for the photocatalytic oxidation of a substrate selected from: an alkane, a cycloalkane, an aromatic hydrocarbon or an alkyl aromatic hydrocarbon comprising contacting the substrate with a θ-type metallacarborane catalyst and an oxidising agent selected from air, O₂ or Na₂S₂O₈ in water, at room temperature and under UV radiation.

## Description

The invention relates to a process for oxidation alkanes and aromatic hydrocarbons to form alcohols. More particularly, the invention relates to a process for the photocatalytic oxidation of the said substrates in presence of θ-type metallacarborane catalysts in water and mild conditions.

Thus, this invention could be framed in the field of synthetic chemistry.

### BACKGROUND ART

The development of methods for the oxidative transformation of inert C-H bonds into more reactive functional groups or involving changes in physical properties, such as generating alcohols, esters, ethers, amines, halides, carbon-carbon bonds is a key aspect of current research and necessary for sustainable development, avoiding as much as possible the extraction of hydrocarbons. It should be noted that C-H bonds are typically strong and kinetically inert, therefore, strong conditions are often required to achieve the activation of such bond, and this causes chemoselective oxidation to be challenging because overoxidation is highly thermodynamically favourable.

Current research is focused on addressing all these challenges and developing new chemical methods for the chemo-, regio- and stereoselective functionalization of C-H bonds in the context of complex organic molecules, in which transition metals are used as catalysts.

Regarding cases of high industrial interest, such as the oxidation of the C-H bond of methane, benzene or mixed alkyl/aromatic hydrocarbons, it is observed that the oxidation of simple alkanes and arenes (such as methane and benzene), in a single step, is a problem of strategic importance worldwide, as natural gas (which is >90% methane) is becoming the precursor for carbon-containing chemicals and liquid fuels. Currently, methane derivatization is achieved by steam reforming to obtain synthesis gas, followed by the Fisher Tropsch process (to access higher alkanes) or methanol synthesis. However, many large chemical companies have found this two-step sequence too costly and inefficient for long-term application. Direct conversion of benzene to phenol is another important industrial goal, as the currently practiced phenol synthesis (the "cumene process") is energy intensive and low yielding.

Some strategies proposed in the state of the art to oxidise C-H bonds, are found, for example, in next document: Joel Rosenthal et al. J. Am. Chem. Soc. 2006, 128, 20, 6546-6547, which discloses the catalytic oxidation of C-H bonds of hydrocarbons by the electron-deficient Pacman porphyrin, (DPDF)Fe₂O, using visible light and molecular oxygen as the terminal oxidant and oxygen atom source. Also, next document: Junghyun Hong et al. ACS Catal. 2013, 3, 9, 2154-2157, it is disclosed a new selective hydrocarbon oxidation heterogeneous catalyst obtained by tethering an iron-coordinated cavitand to the surfaces of a SBA-15 mesoporous material. The resulting material was shown to catalyse the oxidation of cyclic hydrocarbons at room temperature and to be quite robust and easily recyclable.

Taking into account the usefulness and necessity of a method to efficiently oxidise inert C-H bonds in mild conditions, the present invention propose a new process for the transformation of alkanes and aromatic hydrocarbons into their respective alcohols using θ-type metallacarboranes as catalysts. This new process means an advantageous alternative to the known methods of the state of the art.

The compounds θ-type metallacarboranes (also called, metallabisdicarbollides) are anionic compounds with formula M'[M(*m*,*n*-C₂B₉H_{11-y}X_{y})₂], where M' is the counterion, M is a transition metal (e.g. Cr, Fe, Co, Ni, Ru, among others) or a main group metal (e.g. Al, among others); X is halogen, alkyl, chalcogen, or any other substituent; m and n are numbers indicating the position of the carbon atoms in the cluster; and y is the number of non-hydrogen substituents.

Metallacarboranes are part of a broad family of carborane species that contain in their structures one or more transition metals or lanthanides (R. N. Grimes. Coord. Chem. Rev. 2000, 200, 773-811; N. Hosmane and J. Maguire. Comprehensive Organometallic Chemistry III. 2007, 3, 175-264). The metallabisdicarbollides are the metallacarboranes more studied. They are formed by two dianionic dicarbollide clusters with formula [7,8-C₂B₉H₁₁]²⁻ as ligands with a central transition metal which is located endo-cluster.

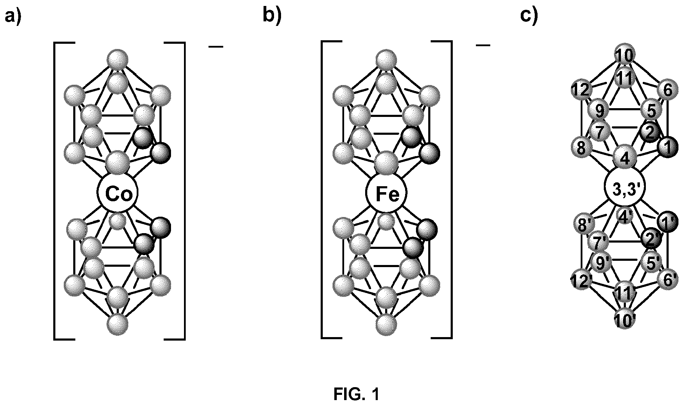

The first metallabisdicarbollide was synthesized by M. F Hawthorne et al. (M. F. Hawthorne, D. C. Young and P. A. Wegner. J. Am. Chem. Soc., 1965, 87, 1818-1819), [3,3'-Fe(1,2-C₂B₉H₁₁)₂]⁻, named ferrabis(dicarbollide), [o-FESAN]- or simply FESAN (Figure 1b). Sometime later, the same authors synthetized a similar complex but based on cobalt, [3,3'-Co(1,2-C₂B₉H₁₁)₂]⁻, named cobaltabis(dicarbollide), [*o*-COSAN]or simply COSAN (Figure 1a)( M. F. Hawthorne and T. D. Andrews. Chem. Commun, , 1965, 443-444).

In COSAN and FESAN the Co³⁺ and Fe³⁺ metal ions are linked to two dicarbollide ligands each one with two negative charges, resulting in a global negative charge delocalized in the overall molecule. This negative charge is delocalized throughout the volume of the molecule leading to a low charge density (C. Masalles, S. Borr6s, C. Viñas and F. Teixidor. Adv. Mater., 2000, 12, 1199-1202) and thanks to the high molecular volume allows to the molecule have a high thermal and chemical stability (C. Viñas, S. Gómez, J. Bertran, F. Teixidor, J.F. Dozol, H. Rouquette, H, Chem. Commun. 1998, 2, 191-192; C. Viñas, S. Gómez, J. Bertran, F. Teixidor, J.F. Dozol, H. Rouquette, H, Inorg. Chem. 1998, 37(14), 3640-3643), global aromaticity (J. Poater, C. Viñas, I. Bennour, s.E. Gordils, M. Solà, F. Teixidor, J. Am. Chem. Soc. 2020, 142(20), 9396-9407) as well as amphiphilic properties (P. Bauduin, S. Prevost, P. Farràs, F. Teixidor, O. Diat and T. Zemb. Angew. Chem. Int. Ed., 2011, 50, 5298-5300), and diverse conformational water/polar medium behaviour (E. J. Juárez-Pérez, R. Núñez, C. Viñas, R. Sillanpää and F. Teixidor. Eur. J. Inorg. Chem, 2010, 2385-2392; D. C. Malaspina, C. Viñas, F. Teixidor and J. Faraudo. Angew. Chem. Int. Ed., 2020, 59, 3088-3092).

### SUMMARY OF THE INVENTION

The inventors have found that the θ-type metallacarboranes are useful as photoredox catalysts for the generation of alcohols or phenols from the corresponding alkane/benzene derivative in a single step, by UV light activation of the metallacarborane. The reaction takes place in water, at room temperature, the yields are between 90-100% and the catalyst can be in molecular form or heterogenized on magnetic particles or adsorbed on solids.

Then, a first aspect of the invention refers to a process (process of the invention) for the photocatalytic oxidation of a set of substrates selected from: alkanes, cycloalkanes, aromatic hydrocarbons or alkyl aromatic hydrocarbons comprising: contacting the substrate with a θ-type metallacarborane catalyst of formula M'[M(C₂B₉H_{11-y}X_{y})₂], wherein:
M' is a counterion selected from: Na⁺, Li⁺, H⁺, K⁺ and [NH₄]⁺ that make the metallacarborane soluble in water (as described in I. Fuentes, A. Andrio, F. Teixidor, C. Viñas, V. Compañ, Phys. Chem. Chem. Phys. 2017, 19, 15177-151869; A. Zaulet, F. Teixidor, P. Bauduin, O. Diat, P. Hirva, A. Ofori, C. Viñas J. Organomet. Chem. 2018, 865, 214-225; M. Tarres, C. Viñas, P. Gonzalez-Cardoso, M. M. Hanninen, R. Sillanpää, V. Dordovic, M. Uchman, F. Teixidor, P. Matejcek, Chem. Eur. J. 2014, 20, 6786 - 6794),
M is a trivalent cation of a metal selected from Cr, Fe, Co, Ni, Ru and Al,
X is H, halogen, chalcogen or alkyl, preferably a C₁-C₁₀ alkyl,
y is an integer selected from 0 to 11,
and an oxidising agent selected preferably from air, O₂ or Na₂S₂O₈, in water, at room temperature and under UV radiation.

In the formula M'[M(C₂B₉H_{11-y}X_{y})₂], the position of the trivalent cation M in the θ-type metallacarborane as well as the position of the carbon atoms in the cluster of the θ-type metallacarborane could be indicated. The position could be indicated by adding the corresponding position numbers just before M and C, respectively, in the formula. In the vast majority of the situations, the position of M is 3,3' or 2,2' depending on the position of C atoms in the cluster that could be from 1,2 to 1,11. That is, one C atom would occupy position 1 if it is adjacent to M and the other carbon atom would occupy any position from 3 to 11. What it is clear is that M merges the two icosahedra that share one vertex occupied by M. In the odd case that the metal and at least one carbon are not adjacent the numbering changes.

Herein, room temperature is considered a temperature between 19 to 25°C.

In a preferred embodiment, the catalyst is selected from Na[3,3'-Co(1,2-C₂B₉H₁₁)₂] (also referred as Na[*o*-COSAN]) and Na[3,3'-Fe(1,2-C₂B₉H₁₁)₂] (also referred as Na[o-FESAN]). The numbers "3,3' " of the formulas indicate the position of the metal (Co or Fe) in the θ-type metallacarborane. The numbers "1,2" indicates the position of the carbon atoms in the cluster of the θ-type metallacarborane.

These metallabisdicarbollides are prepared by metathesis reaction, e.g. cationexchange resin as described in the prior art (I. Fuentes, A. Andrio, F. Teixidor, C. Viñas, V. Compañ, Phys. Chem. Chem. Phys. 2017, 19, 15177-151869), from the water-insoluble Cs[*o*-COSAN] that is commercially available from i.e Katchem or Cs[o-FESAN] that is synthesised (M. F. Hawthorne, D. C. Young and P. A. Wegner. J. Am. Chem. Soc., 1965, 87, 1818-1819), respectively. In addition, recently, C. Viñas *et al.* have published a fast and very efficient green synthesis for this kind of metallacarboranes that does not require any solvent (I. Bennour, A. M. Cioran, F. Teixidor and C. Viñas. Green Chem. 2019, 21, 1925-1928.).

Then, metallacarboranes used in the present invention can be synthetized by following any of the procedures described in the prior art to obtain them.

In a preferred embodiment, when air or O₂ are the oxidising agents, they are used at a pressure of 1 atm. However, higher pressures could be used, for example between 1 and 5 atm, or even higher.

In a preferred embodiment, the UV radiation has a wavelength (λ) between 200 and 400 nm. More preferably, the λ of the UV radiation is selected from: 253.7, 300, 352 or 368 nm and even more preferably, 300 or 352 nm.

In a preferred embodiment, the power of each lamp for the UV radiation is of 2.2 W.

In a preferred embodiment, the concentration of the substrate in the water solution is between 1.5×10⁻² and 0.3 M, more preferably, between 1.5×10⁻² and 2.5×10⁻¹M. Even more preferred concentrations of the substrate are selected from 1.5×10⁻²M, 4.5x10-²M and 2.25×10⁻¹ M.

In a preferred embodiment, the molar ratio catalyst: substrate is between 1:1000 and 1:30000. More preferably, the molar ratio catalyst: substrate is selected from 1:1000, 1:10000 and 1:30000.

In a preferred embodiment, if the solid oxidant Na₂S₂O₈ is used, the molar ratio catalyst: substrate: oxidant is between 1:1000:2000 and 1:30000:60000. More preferably, the molar ratio catalyst: substrate: oxidant is selected from 1:1000:2000, 1:10000:20000 and 1:30000:60000.

In a preferred embodiment, the process is carried out at pH 7. A base, such as K₂CO₃, NaOH or Et₃N can be added to maintain said pH. Compound K₂CO₃ is the most preferred since it is more economical and easier to handle.

A "base", a chemical species that donates electrons, accepts protons, or releases hydroxide (OH⁻) ions in aqueous solution.

In a preferred embodiment, additives, e.g. surfactants among other can be added to the mixture of reaction.

In a preferred embodiment, the reaction time is between 1h and 8h.

The alcohols obtained in the procedure of the present inventions can be monoalcohols, dialcohols or even polyalcohols. The skilled person could make variations within the scope of the described process with no difficulty, for example in the time of reaction, the concentration of the substrate or the catalyst used, to obtain the desired product.

The product of reaction can be isolated by extracting the reaction mixture with an organic solvent, such as dichloromethane or diethyl ether and then, evaporating the organic solvent under reduced pressure.

The term "alkane" refers to a branched or straight hydrocarbon chain, containing only single carbon-carbon bonds. Preferably the alkane used in the process of the present invention as starting material has a chain having 4 to 20 carbons (C₄-C₂₀ alkane). The alkane can be (including branched or straight isomers when possible), for example, methane, propane, butane, pentane, hexane, heptane, octane and so on. In a preferred embodiment, the alkane is n-hexane.

The term "cycloalkane", as used herein, refers to saturated cyclic hydrocarbons having from 3 to about 10 carbon atoms (C₃-C₁₀ cycloalkane), more usually from about 5 to about 8 carbon atoms (C₅-C₈ cycloalkane). Non-limiting examples of cycloalkanes include cyclopentane, cyclohexane, cycloheptane, and cyclooctane. The term "cycloalkane" also includes, according to the present invention, saturated cyclic hydrocarbons having from 3 to about 10 carbon atoms, more usually from about 5 to about 8 carbon atoms, having one or more alkyl substituents with a number of carbon atoms preferably between 1 to 8. In a preferred embodiment, the cycloalkane is cyclohexane.

The term "aromatic hydrocarbon" is intended to mean an organic compound consisting of one or several aromatic cycles, that is, unsaturated cycles, having 4n+2 delocalized pi electrons (fused or linked together by a covalent bond). Examples are benzene, naphthalene and anthracene. In a preferred embodiment, the aromatic hydrocarbon is benzene.

The term "alkyl aromatic hydrocarbon" is intended to mean an "aromatic hydrocarbon" as defined above and having one or several alkyl substituents, preferably one or several C₁-C₅ alkyl substituents, also referred to as side chains. Examples of "alkyl aromatic hydrocarbons" are *o-, m*-or *p*-xylene, hemimellitene, mesitylene, prehnitene, isodurene, durene, ethylbenzene, cumene and *o-,m-* or *p*-cymene In a preferred embodiment, the alkyl aromatic hydrocarbon is toluene.

The term "alkyl" refers to a branched, unbranched, and saturated hydrocarbon radical, including, but not limited to, methyl, ethyl, propyl, butyl, isopropyl, isobutyl, tertiary butyl, pentyl, hexyl and the like.

The term "halogen" used herein refers to fluorine, chlorine, bromine or iodine.

The term "chalcogen" used herein refers to oxygen, selenium or sulfur.

In a preferred embodiment, the substrate is an alkane, more preferably, n-hexane, and the reaction is carried out contacting the substrate with Na[3,3'-Co(1,2-C₂B₉H₁₁)₂] and an oxidizing agent selected from air, O₂ or Na₂S₂O₈ in water, at room temperature and under UV radiation for 4h to 8h. More preferably, the concentration of the substrate is between 1.5×10⁻² and 2.5×10⁻¹ M. Under these conditions, when n-hexane is used, the majority product is a monoalcohol if the reaction time is 4 h, while the majority product is a dialcohol is the reaction time is 8h.

In a preferred embodiment, the substrate is an alkane, preferably n-hexane, and the reaction is carried out contacting the substrate with Na[3,3'-Fe(1,2-C₂B₉H₁₁)₂] and an oxidizing agent selected from air, O₂ or Na₂S₂O₈, preferably O₂, in water, at room temperature and under UV radiation for 8h. More preferably, the concentration of the substrate is between 1.5×10⁻² and 2.5×10⁻¹ M. Under these conditions, when n-hexane is used, the majority product is a dialcohol.

In a preferred embodiment, the substrate is a cycloalkane, preferably cyclohexane, and the reaction is carried out contacting the substrate with Na[3,3'-Co(1,2-C₂B₉H₁₁)₂] or Na[3,3'-Fe(1,2-C₂B₉H₁₁)₂] and an oxidizing agent selected from O₂ or Na₂S₂O₈ in water, at room temperature and under UV radiation for 8h. More preferably, the concentration of the substrate is between 1.5×10⁻² and 2.5×10⁻¹M. Depending on the conditions used, the main product is cyclohexanol or 1,4-ciclohexanediol.

In a preferred embodiment of the method of the present invention, the substrate is an aromatic hydrocarbon, preferably benzene.

In a preferred embodiment, the substrate is an aromatic hydrocarbon, preferably benzene, and the reaction is carried out contacting the substrate with Na[3,3'-Co(1,2-C₂B₉H₁₁)₂] and an oxidizing agent selected from air, O₂ or Na₂S₂O₈ in water, at room temperature and under UV radiation for 1 to 4 h. This reaction allows to obtain phenol with high yield and selectivity. Preferably, if the reaction is carried out with O₂ as oxidising agent (preferably, at 1 atm) and the concentration of the substrate used is 2.25×10⁻¹ M, phenol can be obtained in 1h with a yield of 98% and a selectivity above 99%. Other applicable concentrations are in the range 1,5×10⁻² M-0.3M. When Na₂S₂O₈ is used and/or concentrations of the substrate between 1.5×10⁻² and 4.5×10⁻² M are used, phenol is obtained in 4h.

In a preferred embodiment, the substrate is an aromatic hydrocarbon, preferably benzene, and the reaction is carried out contacting the substrate with Na[3,3'-Co(1,2-C₂B₉H₁₁)₂] and an oxidizing agent selected from air, O₂ or Na₂S₂O₈ in water, at room temperature and under UV radiation for 8h. These conditions lead to di- or polyalcohols derived from benzene as majority products.

In a preferred embodiment, the substrate is an aromatic hydrocarbon, preferably benzene, and the reaction is carried out contacting the substrate with Na[3,3'-Fe(1,2-C₂B₉H₁₁)₂] and an oxidizing agent selected form, O₂ or Na₂S₂O₈ in water, at room temperature and under UV radiation for 4 h to 8h. These conditions lead to di- or polyalcohols derived from benzene as majority products.

In a preferred embodiment, the substrate is an alkyl aromatic hydrocarbon, preferably toluene, wherein reaction is carried out contacting the substrate with Na[3,3'-Co(1,2-C₂B₉H₁₁)₂] or Na[3,3'-Fe(1,2-C₂B₉H₁₁)₂] and an oxidizing agent is Na₂S₂O₈ in water, at room temperature and under UV radiation for 4h to 8 h. These conditions lead to products such as benzyl alcohol, benzoic acid, phenylmethanediol and 4-hydroxybenzoic acid.

The process of the present invention presents many advantages with respect to the known methods of the state of the art to oxidise alkanes and aromatic hydrocarbons. Some of the advantages are highlighted below:
- It does not require high temperatures, high pressures, the use of organic solvents or high-cost catalysts. Contrary to that, the process of the present invention requires mild conditions, such as room temperature and atmospheric pressure, and the reaction solvent/medium is water. The process of the present invention is considered highly efficient, sustainable and environmentally friendly.
- The compound Cs[3,3'-Co(1,2-C₂B₉H₁₁)₂] (immediate precursor of the catalyst Na[3,3'-Co(1,2-C₂B₉H₁₁)₂] used in the examples of the present invention) can be purchased since it is commercially available, which gives the security of using a pure catalyst with guarantees. It should be noted that the catalyst is dissolved in water and is capable of dispersing the organic molecules, something totally new.
- The direct oxidation of the C-H bond in benzene with an oxidising agent such as O₂ to produce phenol is the "dream" reaction for the chemical industry, and this oxidation can be achieved in a few hours with the process of the present invention.
- It can be used for the production of methanol, which is currently an alternative as a transportation fuel. For example, the process can be adapted to carry out onsite oxidation of farm-generated methane to methanol, producing an additional benefit to the farmer, greatly reducing methane emission to the atmosphere and producing a liquid fuel.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention.

### DESCRIPTION OF THE DRAWINGS

**Fig. 1.** a) [*o*-COSAN]- and b) [*o*-FESAN]- anion structures, B-H (light grey), C-H (dark grey) c) metallabisdicarbollide with the numbered atom vertexes.

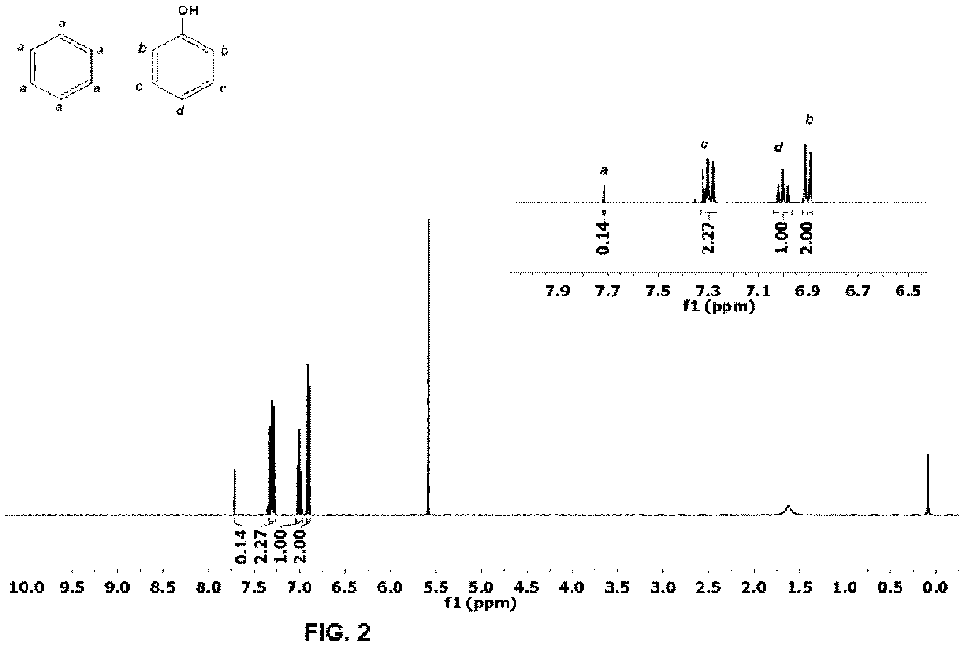

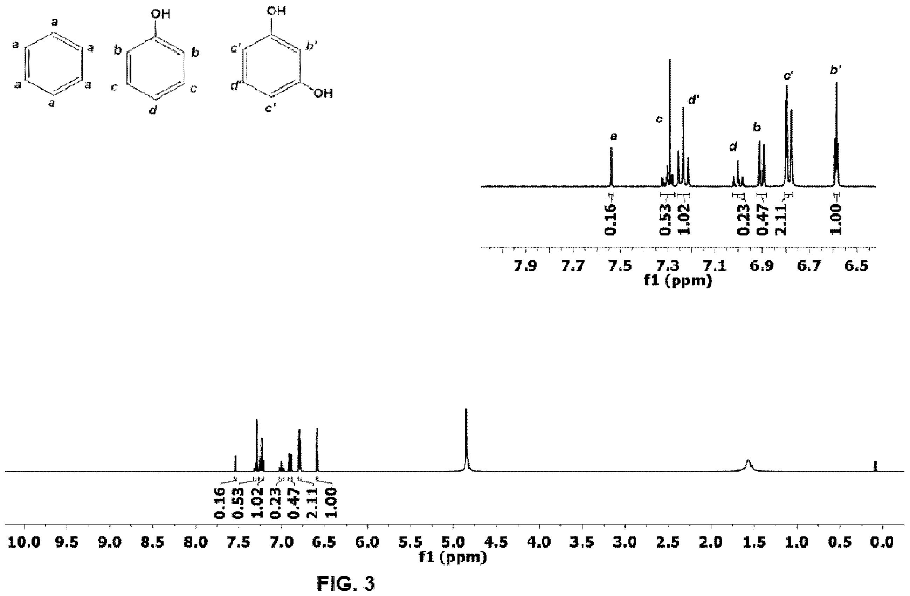

**Fig. 2****.** ¹H NMR spectrum (CDCl₃) corresponding to the photooxidation of benzene, using Na[3,3'-Co(1,2-C₂B₉H₁₁)₂] as catalyst. Conditions: Na[3,3'-Co(1,2-C₂B₉H₁₁)₂] (7.5×10⁻⁶ M), substrate (2.25×10⁻¹ M), O₂ (1 atm), 5 mL water. Light irradiation 1h.
**Fig, 3****.** ¹H NMR spectrum (CDCl₃) corresponding to the photooxidation of benzene, using Na[3,3'-Fe(1,2-C₂B⁹H₁₁)₂] as catalyst. Conditions: Na[3,3'-Fe(1,2-C₂B₉H₁₁)₂] (7.5×10⁻⁶ M), substrate (2.25×10⁻¹ M), Na₂S₂O₈ (4.50×10⁻¹ M), 5 mL water. Light irradiation 8h.

### Examples

The following examples are provided by way of illustration and are not intended to be limiting of the present invention.

### Example 1: Small scale general process:

In a quartz tube, a water solution (5 mL at pH 7) containing the corresponding photoredox catalyst, e.g. Na[3,3'-Co(1,2-C₂B₉H₁₁)₂] or Na[3,3'-Fe(1,2-C₂B₉H₁₁)₂], the substrate (alkane, arene or alkyl aromatic compound) and the oxidant (e.g. Na₂S₂O₈, air or O₂) was exposed to UV light (2.2 W, λ= 253.7 or 300 or 352 or 368 nm) for different times ranging from one to eight hrs. In the set of experiments, a base (e.g. K₂CO₃) was added to water in order to control the pH = 7. For each experiment, light illumination was supplied by a light reactor with fourteen lamps that produce UVA light at room temperature. The resulting solutions were extracted with dichloromethane or diethyl ether three times. The solution was dried with anhydrous sodium sulfate and the solvent was evaporated under reduced pressure. To check the reproducibility of the reactions all experiments were carried out three times. The reaction products were quantified and characterized by ¹H NMR spectroscopy using tetramethylsilane (TMS) as internal standard.

Compounds Na[3,3'-Co(1,2-C₂B₉H₁₁)₂] or Na[3,3'-Fe(1,2-C₂B₉H₁₁)₂] were synthetized by following the method described in the prior art (I. Fuentes, A. Andrio, F. Teixidor, C. Viñas, V. Compañ, Phys. Chem. Chem. Phys. 2017, 19, 15177-151869; T. Garcia-Mendiola, V. Bayon-Pizarro, A. Zaulet, F. Pariente, I. Fuentes, C. Viñas, F. Teixidor and E. Lorenzo. Chem.I Sci., 2016, 7, 5786-5797). Briefly, around 200 mg of the starting compound (Cs[3,3'-Co(1,2-C₂B₉H₁₁)₂]) was dissolved in a minimum volume of acetonitrile/water (50:50) and passed repeatedly (4 times) through the previously prepared cationic resin. Before collecting the solution containing the metallacarborane, 50mL of fresh acetonitrile/water (50:50) was added to the column. In a flask, 50 mL were collected, then the solvent was evaporated and the compound was dried in a vacuum. The cationic resin was prepared following the procedure described in the same reference. Briefly, approximately 2/3 of the volume of the column (30cm) was filled with the strongly acidic cationic exchange resin. Before starting, the cationic resin was kept at 24h in 3M HCl to hydrate it. Then, a 150mL solution of HCl 3M was slowly passed through the column to load it with H⁺. To remove the excess HCl, distilled water was quickly passed through the column until neutral pH was reached. When the desired cation was sodium, a solution of 3M NaCl was passed slowly through the column to exchange H+ with Na+ until neutral pH was reached (the change produced HCl). Distilled water was used to rinse the excess NaCl through the column. To know if NaCl was removed, 3 drops of a solution of 100 mM AgNO₃ was added to a small fraction of solution coming out of the column, until a clear solution was observed. Then, 30 mL of acetonitrile/water (50:50) mixture was allowed to flow through the column to set the column's liquid composition.

**Example 1.1 Detailed description for the oxidation of n-HEXANE.** A mixture of 1.5×10⁻² M of n-hexane, 1.5×10⁻⁶ M of Na[o-COSAN] as catalyst, and a cat/substrate ratio of 1:10000 , bubbling O₂ at 1 atm in 5 mL of H₂O (pH = 7) was irradiated under UV light (λ= 352 nm). After 4 hours of irradiation, 1-hexanol was produced in 68% yield (99% of selectivity). When Na[*o*-FESAN] was used as catalyst and UV light (λ= 300 nm) in the same proportion, 1,1-hexanediol was obtained in 89% yield (99% of selectivity) after 8 hours.

**Example 1.2 Detailed description for the oxidation of CYCLOHEXANE.** A mixture of 1.5×10⁻² M of cyclohexane, 1.5×10⁻⁶ M of Na[*o*-COSAN] as catalyst and a cat/substrate ratio of 1:10000, bubbling O₂ at 1 atm in 5 mL of H₂O (pH = 7) was irradiated under UV light (λ= 352 nm). After 8 hours of irradiation, the monosubstituted cyclohexanol was produced in 92% yield (99% of selectivity). On the other hand, when Na[o-FESAN] was used as catalyst, Na₂S₂O₈ as oxidant and UV light (λ= 300 nm), a diol, 1,4-cyclohexanediol was produced in 74% yield (76 % of selectivity) after 8 hours of reaction.

**Example 1.3. Detailed description for the oxidation of BENZENE.** When benzene (1.5×10⁻² M) was used as substrate, 1.5×10⁻⁶ M of Na[*o*-COSAN] as catalyst and 3.0×10⁻² M of Na₂S₂O₈ as oxidant. The ratio cat/substrate ratio of 1:10000:20000 was used. Phenol was obtained in 96% yield (99% selectivity) after 4 hours or reaction, whereas resorcinol was obtained as the only product in a 99% yield after extension of the reaction to 8 hours, using only 1.5×10⁻⁶ M of catalyst. Using the same conditions but O₂ as oxidant, 72% of phenol after 4 hours was obtained.

Besides, when Na[o-FESAN] was used as catalyst, hydroquinone was formed in 67% yield and high selectivity (99%) with O₂ as oxidant and 4 hours of reaction. However, with longer times of reaction up to 8 hours, polyhydroxilation was achieved.

**Example 1.4. Detailed description for the oxidation of TOLUENE.** Using 1.5×10⁻² M of toluene as substrate, 1.5×10⁻⁵ M of Na[*o*-COSAN] as catalyst and 3.0×10⁻² M of Na₂S₂O₈ as oxidant. The ratio cat/substrate ratio of 1:1000:2000 was used. The formation of benzyl alcohol in 76% yield and high selectivity (96%) after 8 hours was achieved. In the case of using Na[o-FESAN], benzoic acid was produced in a 59% yield with the same selectivity after 8 hours of reaction. Remarkably, polyalcohols as phenylmethanediol was obtained in 87% yield and 88 % of selectivity after 4 hours of reaction using 1.5×10⁻⁶ M of Na[*o*-FESAN]. In addition, 4-hydroxybenzoic acid* was produced in 73% yield and the same selectivity using Na[o-COSAN] and 8 hours of reaction.
* This result indicated that the toluene has been hydroxylated in one aromatic carbon of the phenyl ring after the methyl group has been oxidized to acid.

### Example 2. Scaling up the substrate concentration experiments

For scaling up the reactions, a higher concentration of substrate (e.g. 4.5×10⁻² M or 2.25×10⁻¹M) and a cat/substrate/oxidant ratio of 1:30000:60000 were used. In these experiments Na[*o*-FESAN] and Na[*o*-COSAN], and Na₂S₂O₈, O₂ or air as oxidants were used. O₂ or air are used in excess without controlling the proportion.

**Example 2.1. Detailed description for the oxidation of ALKANES.** Using hexane 4.5×10⁻² M as substrate and Na[o-COSAN] in 1.5×10⁻⁶ M and Na₂S₂O₈ in 9.0×10⁻² M as oxidant, 1-hexanol in 43% yield was obtained after 4 hours, but at 8 hours, the 1,1-hexanediol was produced in 67% yield with the same selectivity. When cyclohexane was used as substrate in the same conditions, 83% of 1,4-cyclohexanediol with a high selectivity of 87% was obtained after 8 hours.

**Example 2.2. Detailed description for the oxidation BENZENE.** Starting from benzene 4.5×10⁻² M as substrate and Na[*o*-COSAN] as catalyst in 1.5×10⁻⁶ M, phenol was obtained in a 72% yield and the same selectivity after 4 hours of reaction and using Na₂S₂O₈ as oxidant. Whereas resorcinol was produced in 57% with moderate selectivity after 8 hours of reaction.

By increasing the concentration of substrate (e.g. 2.25×10⁻¹ M), phenol was formed in 47% yield and 66% selectivity after 4 hours of reaction and using Na[*o*-COSAN] in 7.5×10⁻⁶ M as catalyst and Na₂S₂O₈ in 4.5×10⁻¹ M as oxidant. On the contrary, when the catalyst was changed to Na[o-FESAN], resorcinol was obtained in 79% yield (82% selectivity) after 8h of reaction.

Finally, for Benzene as substrate, the most prominent results were obtained using Na[*o*-COSAN] as catalyst and O₂ or air as oxidants, from where phenol was obtained as the only product after 1 hour in a 98% yield (99% selectivity) using O₂ as oxidant or pyrocatechol as the only product after 4 hours in a 96 yield (99 % selectivity) using air as oxidant. If the reaction is maintained for 4 hours, resorcinol is formed in 76% yield and the same selectivity, and 68% after 8 hours, using O₂ as oxidant and other products (pyrocatechol and hydroquinone) are released in lower yield in these conditions.

In following Table 1, the more remarkable results described above are also outlined.

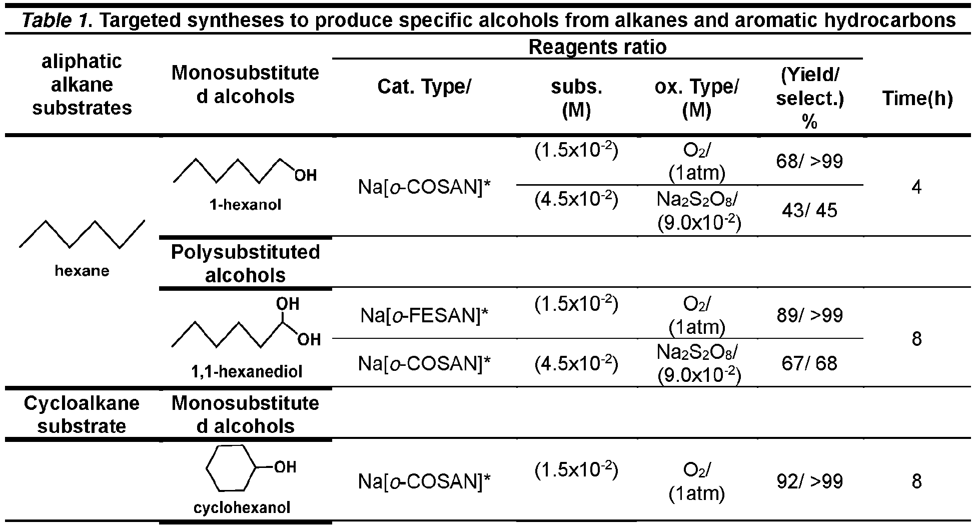

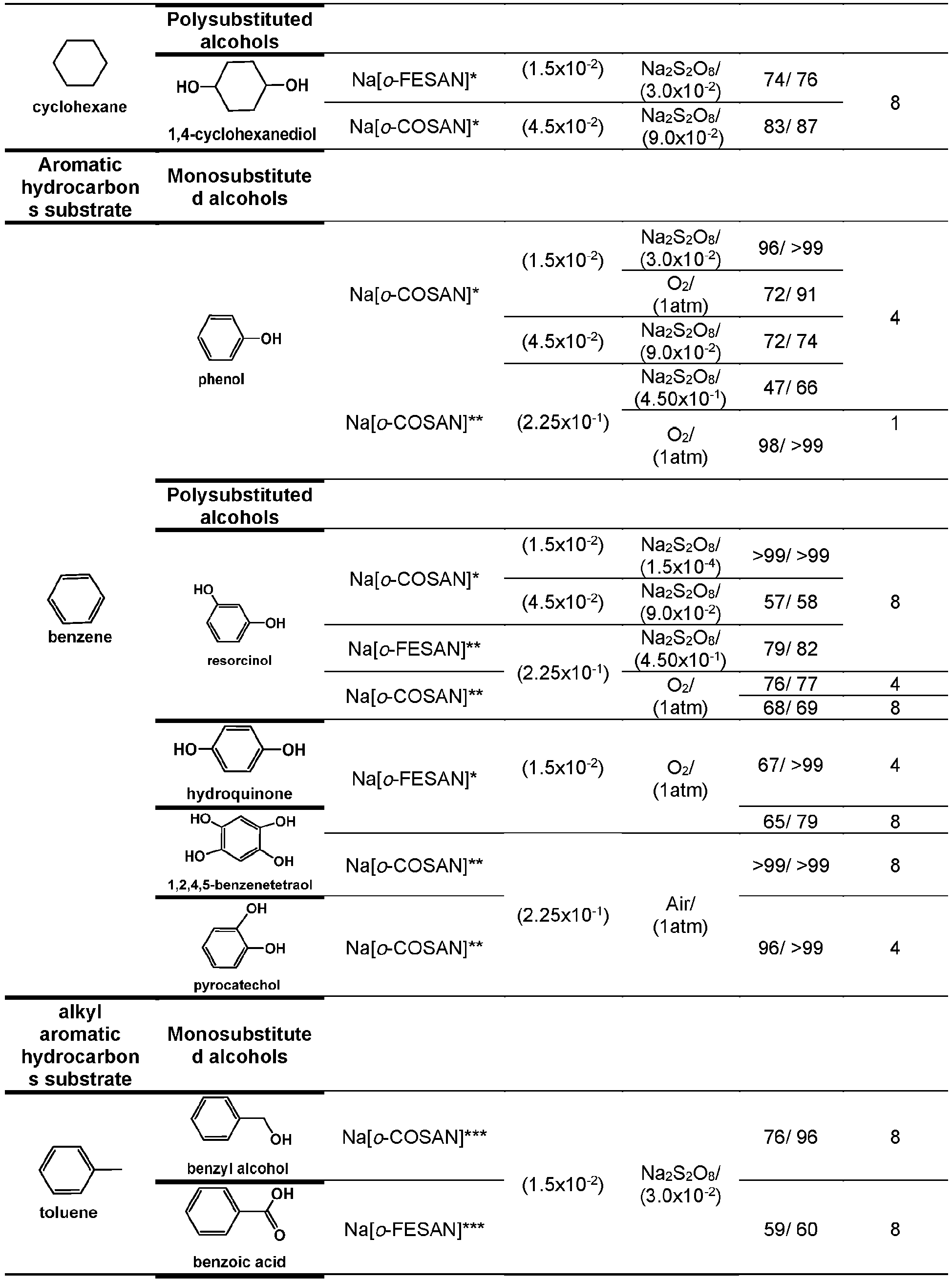

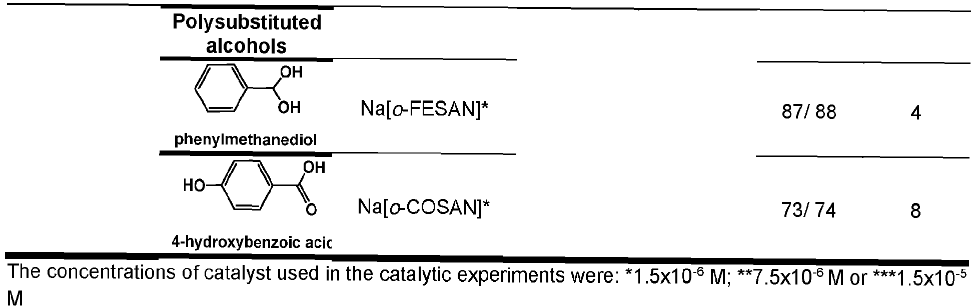

## Claims

1. Process for the photocatalytic oxidation of a substrate selected from: an alkane, a cycloalkane, an aromatic hydrocarbon or an alkyl aromatic hydrocarbon comprising: contacting the substrate with a θ-type metallacarborane catalyst of formula M'[M(C₂B₉H_{11-y}X_{y})₂], wherein:
M' is a counterion selected from: Na⁺, Li⁺, H⁺, K⁺ and [NH₄]⁺
M is a trivalent cation of a metal selected from Cr, Fe, Co, Ni, Ru and Al,
X is H, halogen, chalcogen or alkyl, preferably C₁-C₁₀ alkyl,
y is an integer selected from 0 to 11,
and an oxidising agent in water, at room temperature and under UV radiation.

2. The process, according to claim 1, wherein the catalyst is selected from Na[3,3'-Co(1,2-C₂B₉H₁₁)₂] and Na[3,3'-Fe(1,2-C₂B₉H₁₁)₂].

3. The process, according to any of previous claims 1 or 2, wherein the concentration of the substrate in the water solution is between 1.5×10⁻² M and 0.3 M.

4. The process, according to any of previous claims 1 to 3, wherein the molar ratio catalyst:substrate is between 1:1000 and 1:30000.

5. The process, according to any of previous claims 1 to 4, , wherein the oxidising agent is selected from air, O₂ or Na₂S₂O₈.

6. The process, according to any of previous claims 1 to 5, wherein the oxidising agent is Na₂S₂O₈ and the molar ratio catalyst:substrate:oxidant is between 1:1000:2000 and 1:30000:60000.

7. The process, according to any of previous claims 1 to 6, wherein reaction is carried out at pH 7.

8. The process, according to any of previous claims 1 to 7, wherein the reaction time is between 1h and 8h.

9. The process, according to any of previous claims 1 to 8, wherein the substrate is an alkane and the reaction is carried out contacting the substrate with Na[3,3'-Co(1,2-C₂B₉H₁₁)₂] and an oxidizing agent selected from air, O₂ or Na₂S₂O₈ in water, at room temperature and under UV radiation for 4h or 8h.

10. The process, according to any of previous claims 1 to 8, wherein the substrate is an alkane and the reaction is carried out contacting the substrate Na[3,3'-Fe(1,2-C₂B₉H₁₁)₂] and an oxidizing agent selected from air, O₂ or Na₂S₂O₈ in water, at room temperature and under UV radiation for 8h.

11. The process, according to any of previous claims 1 to 8, wherein the substrate is a cycloalkane and the reaction is carried out contacting the substrate with Na[3,3'-Co(1,2-C₂B₉H₁₁)₂] or Na[3,3'-Fe(1,2-C₂B₉H₁₁)₂] and an oxidizing agent selected from air, O₂ or Na₂S₂O₈ in water, at room temperature and under UV radiation for 8h.

12. The process, according to any of previous claims 1 to 8, wherein the substrate is an aromatic hydrocarbon, preferably benzene.

13. The process, according to claim 12, wherein reaction is carried out contacting the substrate with Na[3,3'-Co(1,2-C₂B₉H₁₁)₂] and an oxidizing agent is air, O₂ or Na₂S₂O₈ in water, at room temperature and under UV radiation for 1 to 4 h.

14. The process, according to claim 12, wherein reaction is carried out contacting the substrate with Na[3,3'-Fe(1,2-C₂B₉H₁₁)₂] and an oxidizing agent is air, O₂ or Na₂S₂O₈ in water, at room temperature and under UV radiation for 4 or 8h.

15. The process, according to any of previous claims 1 to 8, wherein the substrate is alkyl aromatic hydrocarbon, preferably toluene, wherein reaction is carried out contacting the substrate with Na[3,3'-Fe(1,2-C₂B₉H₁₁)₂] or Na[3,3'-Co(1,2-C₂B₉H₁₁)₂] and an oxidizing agent is Na₂S₂O₈ in water, at room temperature and under UV radiation for 4h or 8 h.
